(19) Europäisches Patentamt
European Patent Office
Office européen des brevets

(11) **EP 3 424 539 A1**

## (12) EUROPEAN PATENT APPLICATION

(43) Date of publication:
**09.01.2019 Bulletin 2019/02**

(51) Int Cl.:
***A61L 9/01*** *(2006.01)*     ***C08L 23/06*** *(2006.01)*

(21) Application number: **17180038.6**

(22) Date of filing: **06.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **The Procter & Gamble Company Cincinnati, OH 45202 (US)**

(72) Inventors:
- **HORENZIAK, Steven Anthony Cincinnati, 45217 (US)**
- **FRANKENBACH, Gayle Marie Cincinnati, 45217 (US)**
- **HOLLINGSHEAD, Judith Ann Cincinnati, 45217 (US)**

(74) Representative: **Yorquez Ramirez, Maria Isabel et al**
**Procter & Gamble**
**Technical Centres Limited**
**Whitley Road**
**Longbenton**
**Newcastle upon Tyne NE12 9TS (GB)**

### (54) MALODOR REDUCTION COMPOSITIONS

(57) The present invention relates to malodor reduction compositions and methods of making and using same. The malodor reduction compositions are suitable for use in a variety of applications, including use in consumer products, for example, air freshening compositions, laundry detergents, fabric enhancers, surface cleaners, beauty care products, dish care products, diapers, feminine protection articles, and plastic films for garbage bags. Such malodor control technologies do not unduly interfere with the scent of the perfumed or unperfumed situs that is treated with the malodor control technology.

**EP 3 424 539 A1**

**Description**

FIELD OF THE INVENTION

The present invention relates to malodor reduction compositions and methods of making and using same.

BACKGROUND OF THE INVENTION

Unscented or scented products are desired by consumers as they may be considered more natural and discreet than scented products. Manufacturers of unscented or scented products for controlling malodors rely on malodor reduction ingredients or other technologies (e.g. filters) to reduce malodors. However, effectively controlling malodors, for example, amine-based malodors (e.g. fish and urine), thiol and sulfide-based malodors (e.g. garlic and onion), $C_2$-$C_{12}$ carboxylic acid based malodors (e.g. body and pet odor), indole based malodors (e.g. fecal and bad breath), short chain fatty aldehyde based malodors (e.g. grease) and geosmin based malodors (e.g. mold/mildew) may be difficult, and the time required for a product to noticeably reduce malodors may create consumer doubt as to the product's efficacy on malodors. Manufacturers often incorporate scented perfumes to help mask these difficult malodors.

Unfortunately, malodor control technologies typically cover up the malodor with a stronger scent and thus interfere with the scent of the perfumed or unperfumed situs that is treated with the malodor control technology. Thus, limited nature of the current malodor control technologies is extremely constraining. Thus, what is needed is a broader palette of malodor control technologies so the perfume community can deliver the desired level of character in a greater number of situations/applications. Surprisingly, Applicants recognized that in addition to blocking a malodor's access to a sensory cell, to achieve the desired goal, a malodor control technology must leave such sensor cell open to other molecules, for example scent molecules. Thus, the malodor control technologies disclosed herein do not unduly interfere with the scent of the perfumed or unperfumed situs that is treated with the malodor control technology.

SUMMARY OF THE INVENTION

The present invention relates to malodor reduction compositions and methods of making and using same. The malodor reduction compositions are suitable for use in a variety of applications, including use in consumer products, for example, air freshening compositions, laundry detergents, fabric enhancers, surface cleaners, beauty care products, dish care products, diapers, feminine protection articles, and plastic films for garbage bags. Such malodor control technologies do not unduly interfere with the scent of the perfumed or unperfumed situs that is treated with the malodor control technology.

DETAILED DESCRIPTION OF THE INVENTION

As used herein "MORV" is the calculated malodor reduction value for a subject material. A material's MORV indicates such material's ability to decrease or even eliminate the perception of one or more malodors. For purposes of the present application, a material's MORV is calculated in accordance with method found in the test methods section of the present application.

As used herein "consumer product" means baby care, beauty care, fabric & home care, family care, feminine care, health care products or devices. Such products include but are not limited to plastic films garbage bags, storage bags, storage wraps, diapers, bibs, wipes, garments, textiles including sheets and towels, composters; products for and/or methods relating to treating hair (human, dog, and/or cat), including, bleaching, coloring, dyeing, conditioning, wet or dry shampooing, styling, scalp treatments; deodorants and antiperspirants; personal cleansing; cosmetics; skin care including application of creams, lotions, fine fragrances and other topically applied products for consumer use; and shaving products, products for and/or methods relating to treating fabrics, hard surfaces and any other surfaces in the area of fabric and home care, including: air care including air filtration, car care, dishwashing, fabric conditioning (including softening), laundry detergency, laundry and rinse additive and/or care, hard surface cleaning and/or treatment, towel bowl cleaners and other cleaning and/or malodor treatments for consumer, agricultural, industrial or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening.

As used herein "energized system", refers to a system that operates by using an electrical and/or mechanical energy source such as a battery or electrical wall outlet to emit the malodor reduction composition. Examples of such devices include but are limited to liquid electric pluggable type air freshening devices.

As used herein, "malodor" refers to compounds generally offensive or unpleasant to most people, such as the complex odors associated with bowel movements.

As used herein, "neutralize" or "neutralization" refers to the ability of a compound or product to reduce or eliminate malodorous compounds. Odor neutralization may be partial, affecting only some of the malodorous compounds in each context, or affecting only part of a malodorous compound. A malodorous compound may be neutralized by chemical reaction resulting in a new chemical entity, by sequestration, by chelation, by association, or by any other interaction rendering the malodorous compound less malodorous or non-malodorous.

As used herein, "non-energized" refers to a system that emits a targeted active passively or without the need for an electrical energy source. Handheld aerosol sprayers and traditional trigger/pump sprayers are considered non-energized systems.

As used herein, "odor blocking" refers to the ability of a compound to dull the human sense of smell.

As used herein, "odor masking" refers to the ability of a compound with a non-offensive or pleasant smell that is dosed such that it limits the ability to sense a malodorous compound. Odor-masking may involve the selection of compounds which coordinate with an anticipated malodor to change the perception of the overall scent provided by the combination of odorous compounds.

As used herein, "compostable film" means a film that, when made into a bag, meets the testing specifications found in ASTM D6400.

As used herein, the terms "a" and "an" mean "at least one".

As used herein, the terms "include", "includes" and "including" are meant to be non-limiting.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The benefit agents and perfumes raw materials in this specification can be obtained from suppliers including: International Flavors and Fragrances of New York, NY USA; Givaudan of Vernier Switzerland; Firmenich of Geneva, Switzerland; Symrise of Holzminden, Germany; Kao of Tokyo, Japan; Takasago of Tokyo, Japan; Merck's Sigma Aldrich division of Darmstadt, Germany and Florasynth of Tel-Aviv, Israel.

Compositions and Methods

A composition comprising a malodor reduction material having an MORV of at least 0.5, from about 0.5 to about 10, from 0.5 to about 10, from about 1 to about 10, or from about 1 to about 5 is disclosed.

A composition comprising a malodor reduction material selected from the group consisting of 3,5-dimethyladamantane-1-carboxylic acid; (1R, 6S)-6-(methoxycarbonyl)cyclohex-3-ene-1-carboxylic acid; (E)-1,1,4,4-tetramethoxybut-2-ene;
dimethyl (4R,5R)-2,2-dimethyl-1,3-dioxolane-4,5-dicarboxylate; 1,2,3,4,5,6-hexamethylbenzene; dimethyl (1R,2S)-cyclohex-4-ene-1,2-dicarboxylate and mixtures thereof is disclosed. Preferably, said composition comprises, based on total composition weight, from 0.0001% to 100% of a malodor reduction material selected from the group consisting of 3,5-dimethyladamantane-1-carboxylic acid; (1R,6S)-6-(methoxycarbonyl)cyclohex-3-ene-1-carboxylic acid; (E)-1,1,4,4-tetramethoxybut-2-ene;
dimethyl (4R,5R)-2,2-dimethyl-1,3-dioxolane-4,5-dicarboxylate; 1,2,3,4,5,6-hexamethylbenzene; dimethyl (1R,2S)-cyclohex-4-ene-1,2-dicarboxylate and mixtures thereof, and an adjunct material. Preferably, said composition is a consumer product.

A plastic film said plastic film comprising linear low density polyethylene (LLDPE), low density polyethylene (LDPE), high density polyethylene (HDPE), and/or compostable film and a malodor reduction material selected from the group consisting of 3,5-dimethyladamantane-1-carboxylic acid; (1R,6S)-6-(methoxycarbonyl)cyclohex-3-ene-1-carboxylic acid; (E)-1,1,4,4-tetramethoxybut-2-ene; dimethyl (4R,5R)-2,2-dimethyl-1,3-dioxolane-4,5-dicarboxylate; 1,2,3,4,5,6-hexamethylbenzene; dimethyl (1R,2S)-cyclohex-4-ene-1,2-dicarboxylate and mixtures thereof is disclosed.

A method of controlling malodors comprising contacting a material comprising a malodor with a composition comprising a malodor reduction material selected from the group consisting of 3,5-dimethyladamantane-1-carboxylic acid; (1R,6S)-6-(methoxycarbonyl)cyclohex-3-ene-1-carboxylic acid; (E)-1,1,4,4-tetramethoxybut-2-ene; dimethyl (4R,5R)-2,2-dimethyl-1,3-dioxolane-4,5-dicarboxylate; 1,2,3,4,5,6-hexamethylbenzene; dimethyl (1R,2S)-cyclohex-4-ene-1,2-dicarboxylate and mixtures thereof is disclosed. Preferably, said composition comprises, based on total composition weight, from 0.0001% to 100% of a malodor reduction material selected from the group consisting of 3,5-dimethyladamantane-1-carboxylic acid; (1R,6S)-6-(methoxycarbonyl)cyclohex-3-ene-1-carboxylic acid; (E)-1,1,4,4-tetramethoxybut-2-ene;

dimethyl (4R,5R)-2,2-dimethyl-1,3-dioxolane-4,5-dicarboxylate; 1,2,3,4,5,6-hexamethylbenzene;

dimethyl (1R,2S)-cyclohex-4-ene-1,2-dicarboxylate and mixtures thereof, and an adjunct material. Preferably, said composition is a consumer product.

The use of a composition comprising a malodor reduction material selected from the group consisting of 3,5-dimethyladamantane-1-carboxylic acid; (1R,6S)-6-(methoxycarbonyl)cyclohex-3-ene-1-carboxylic acid; (E)-1,1,4,4-tetramethoxybut-2-ene;

dimethyl (4R,5R)-2,2-dimethyl-1,3-dioxolane-4,5-dicarboxylate; 1,2,3,4,5,6-hexamethylbenzene; dimethyl (1R,2S)-cyclohex-4-ene-1,2-dicarboxylate and mixtures thereof to reduce a malodor is disclosed

A composition according to any preceding embodiment, said malodor reduction composition being a consumer product, said consumer product comprising a total of, based on total consumer product weight, from about 0.0001% to about 100% of one or more of said malodor reduction materials and an adjunct material is disclosed.

Said malodor reduction composition that is a consumer product is a laundry detergent that comprises a total of, based on total consumer product weight, from about 0.0001% to about 10%, in one aspect, from about 0.001% to about 5%, in one aspect, from about 0.01% to about 3%, in one aspect, from about 0.1% to about 2% of one or more of said malodor reduction materials and, a material selected from the group consisting of surfactants, builders, chelating agents, dye transfer inhibiting agents, dispersants, enzymes, and enzyme stabilizers, catalytic materials, bleach activators, a fabric softener active, hydrogen peroxide, sources of hydrogen peroxide, preformed peracids, polymeric dispersing agents, clay soil removal/anti-redeposition agents, brighteners, suds suppressors, dyes, hueing dyes, perfumes, perfume delivery systems, structure elasticizing agents, carriers, structurants, hydrotropes, processing aids, solvents, pigments and mixtures thereof, in none aspect, said laundry detergent comprises an adjunct material selected from the group consisting of an organic acid, in one aspect, citric acid and/ or lactic acid, hydrogenated castor oil, ethoxylated polyethyleneimines, PEI 600 EO 20 and/or PEI 600, an enzyme, a cold water amylase , cold water protease and/ or xyloglucanase.

Said malodor reduction composition that is a consumer product is an air care composition that comprises a total of, based on total consumer product weight, from about 0.001% to about 100%, preferably from 0.01 to 10%, of one or more of said malodor reduction materials and, optionally, one or more materials selected from the group consisting of surfactants, antimicrobial agents, wetting agents, buffering agents, cyclodextrins, propellants, and solvents.

Said malodor reduction composition that is a consumer product is a liquid fabric enhancer that comprises a total of, based on total composition weight, from about 0.05% to about 10% of one or more of said malodor reduction materials and, a fabric softener active selected from the group consisting of a quaternary ammonium compound, a silicone polymer, a polysaccharide, a clay, an amine, a fatty ester, a dispersible polyolefin, a polymer latex and mixtures thereof, said quaternary ammonium compound is selected from the group consisting of bis-(2-hydroxypropyl)-dimethylammonium methylsulphate fatty acid ester, 1,2-di(acyloxy)-3-trimethylammoniopropane chloride., N, N-bis(stearoyl-oxy-ethyl) N,N-dimethyl ammonium chloride, N,N-bis(tallowoyl-oxy-ethyl) N,N-dimethyl ammonium chloride, N,N-bis(stearoyl-oxy-ethyl) N-(2 hydroxyethyl) N-methyl ammonium methylsulfate, 1, 2 di (stearoyl-oxy) 3 trimethyl ammoniumpropane chloride, dicanoladimethylammonium chloride, di(hard)tallowdimethylammonium chloride dicanoladimethylammonium methylsulfate, 1-methyl-1-stearoylamidoethyl-2-stearoylimidazolinium methylsulfate, 1-tallowylamidoethyl-2-tallowylimidazoline, Dipalmethyl Hydroxyethylammoinum Methosulfate and mixtures thereof, said fabric softening active having an Iodine Value of between 0-140, in one aspect, 5-100, in one aspect, 10-80, in one aspect, 15-70, in one aspect 18-25.

Said malodor reduction composition that is a consumer product is a fabric enhancer solid particle or bead composition comprising from 0.05% to about 10% of one or more of said malodor reduction materials and at least about 25% polyethyleneglycol (PEG) 8000.

Said malodor reduction composition that is a consumer product is a soluble unit-dose composition contained within a polyvinyl alcohol (PVA) film.

Said malodor reduction composition that is a consumer product is a fabric softener dryer added sheet composition, said fabric softener dryer added sheet composition comprising from 0.05% to about 10% of one or more of said malodor reduction materials and impregnated onto a non-woven sheet.

Said malodor reduction composition that is a consumer product is a spray-dried laundry detergent powder, said composition comprising from about 0.05% to about 20% of one or more of said malodor reduction materials and at least 10% solids chosen from the following list or combinations thereof: sodium carbonate, sodium sulfate, carboxy methyl cellulose powder, a polymer comprising an acrylate monomer.

Said malodor reduction composition that is a consumer product is a plastic film said plastic film comprising LLDPE, LDPE, HDPE, and/or compostable film, in one aspect, said plastic film comprises about 0.5 mg to about 100 mg of said malodor reduction composition per 20 grams of said plastic film, in one aspect, said malodor reduction composition is present in the amount of about 5 mg to about 30 mg per 20 grams of said plastic film, in one aspect, said malodor reduction composition is present in the amount of about 5 mg to about 15 mg per 20 grams of said plastic film.

Said malodor reduction composition that is a consumer product is an absorbent article said absorbent article comprising a total of, based on total consumer product weight, from about 0.1% to about 75%, in one aspect, from about 1% to about 50%, from about 5% to about 40%, from about 12% to about 30% of one or more of said malodor reduction materials and, wherein the article comprises a topsheet, a backsheet, an absorbent core disposed between the topsheet and the backsheet, and an acquisition layer disposed between the topsheet and the absorbent core, wherein the malodor reduction composition is disposed on one of the backsheet, absorbent core, or acquisition layer, said absorbent article comprises a hot melt adhesive and the hot melt adhesive comprises the malodor reduction composition, the hot melt adhesive adheres the backsheet to the topsheet, the backsheet comprises a nonwoven layer and a film layer, and the hot melt adhesive adheres the backsheet nonwoven layer to the backsheet film layer.

Said malodor reduction composition that is a consumer product is a personal care deodorant composition, said deodorant composition comprising a total of, based on total consumer product weight, from about 0.1% to about 7% of one or more of said malodor reduction materials and, optionally, from about 0.01% to about 75% of an antimicrobial, said antimicrobials are selected from the group consisting of metals, zeolites, metal zeolites, quaternary ammonium (quat) compounds (e.g., cetyl pyridinium chloride, and benzylalkonium chloride), quat bound clays, metal bound clays, polyaspirin. salicylic acid, polyvinyl amines, coal tar, sulfur, whitfield's ointment, castellani's paint, aluminum chloride, gentian violet, octopirox (piroctone olamine), ciclopirox olamine, undecylenic acid and its metal salts, potassium permanganate, selenium sulfide, sodium thiosulfate, glycols, diols, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, citronellic acid, hinokitol, ichthyol pale, Sensiva SC-50, Elestab HP-100, azelaic acid, lyticase, iodopropynyl butylcarbamate (IPBC), triclosan, triclocarban, isothiazalinones and azoles, and combinations thereof, more preferably, hexanediol, triclosan, octyl isothiazalinone metals selected from the group consisting of Zn, Cu, Al, Ti, Sn, Bi, and Ag, metal salts selected from the group consisting of zinc carbonate, copper sulfate, and zinc gluconate, metal pyrithione salts selected from the group consisting of zing pyrithione (ZPT) and copper pyrithione (CuPT), glycols selected from the group consisting of propylene glycol, dipropylene glycol and hexylene glycol and mixtures thereof, said personal care deodorant composition comprises, based on total deodorant weight, from about 10% to about 75% glycol.

Said malodor reduction composition that is a consumer product is a personal care body wash/shampoo composition, said body wash/shampoo comprising a total of, based on total consumer product weight, from about 0.1% to about 7% of one or more of said malodor reduction materials from about 3% to 30% of a surfactant, and, optionally, a micellar phase and/or lamellar phase.

Said malodor reduction composition that is a consumer product is a personal care antiperspirant composition, said antiperspirant composition comprising a total of, based on total consumer product weight, from about 0.1% to about 7% of one or more of said malodor reduction materials and, optionally, from about 1% to about 25% of an aluminum salt antiperspirant active.

Said malodor reduction composition that is a consumer product is an anhydrous antiperspirant composition, said anhydrous antiperspirant composition comprising a total of, based on total consumer product weight, from about 0.1% to about 7% of one or more of said malodor reduction materials and from about 1% to about 25% of an antiperspirant actives selected from the group consisting of astringent metallic salts, inorganic and organic salts of aluminum, zirconium and zinc, as well as mixtures thereof, aluminum halides, aluminum chlorohydrate, aluminum hydroxyhalides, zirconyl oxyhalides, zirconyl hydroxyhalides, and mixtures thereof.

Said malodor reduction composition that is a consumer product is a dish cleaning composition, said dish cleaning composition comprising a total of, based on total consumer product weight, from about 0.1% to about 7% of one or more of said malodor reduction materials.

A method of controlling malodors comprising: contacting the material comprising a malodor with a composition selected from the group consisting of any of the compositions comprising a malodor reduction material disclosed herein and mixtures thereof, said contacting step comprises contacting said material containing a malodor with about 1 mg to about 50 mg, from about 3 mg to 30 mg, or from about 5 mg to about 20 mg of said composition per 20 grams of said material containing a malodor is disclosed.

A method of determining the material's ability to decrease or even eliminate the perception of one or more malodors comprising determining the MORV and/or Universal MORV of said material, in one aspect, determining said MORV and/or Universal MORV by the MORV and Universal MORV method provided in Test Method 1, is disclosed.

Adjunct Materials

While not essential for the purposes of the present invention, the non-limiting list of adjuncts illustrated hereinafter are suitable for use in the instant compositions and may be desirably incorporated in certain aspects of the invention, for example to assist or enhance cleaning performance, for treatment of the substrate to be cleaned, or to modify the aesthetics of the composition as is the case with perfumes, colorants, dyes or the like. The precise nature of these additional components, and levels of incorporation thereof, will depend on the physical form of the composition and the nature of the fabric treatment operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, hueing dyes, perfumes, perfume delivery systems, structure elasticizing agents, carriers, structurants, hydrotropes, processing aids, solvents, pigments and/or fabric softener actives.

As stated, the adjunct ingredients are not essential to Applicants' compositions. Thus, certain aspects of Applicants' compositions do not contain one or more of the following adjuncts materials: surfactants, builders, chelating agents, dye transfer inhibiting agents, dispersants, enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, hueing dyes, perfumes, perfume delivery systems structure elasticizing agents, carriers, hydrotropes, processing aids, solvents, pigments and/or fabric softener actives. However, when one or more adjuncts are present, such one or more adjuncts may be present as detailed below.

Hueing Dye - The liquid laundry detergent composition may comprise a hueing dye. The hueing dyes employed in the present laundry care compositions may comprise polymeric or non-polymeric dyes, organic or inorganic pigments, or mixtures thereof. In one aspect, the hueing dye comprises a polymeric dye, comprising a chromophore constituent and a polymeric constituent. The chromophore constituent is characterized in that it absorbs light in the wavelength range of blue, red, violet, purple, or combinations thereof upon exposure to light. In one aspect, the chromophore constituent exhibits an absorbance spectrum maximum from about 520 nanometers to about 640 nanometers in water and/or methanol, and in another aspect, from about 560 nanometers to about 610 nanometers in water and/or methanol.

Although any suitable chromophore may be used, the dye chromophore is, in one aspect, selected from benzodifuranes, methine, triphenylmethanes, napthalimides, pyrazole, napthoquinone, anthraquinone, azo, oxazine, azine, xanthene, triphenodioxazine and phthalocyanine dye chromophores. Mono and di-azo dye chromophores are may be preferred.

Surfactants - The compositions according to the present invention may comprise a surfactant or surfactant system wherein the surfactant can be selected from nonionic surfactants, anionic surfactants, cationic surfactants, ampholytic surfactants, zwitterionic surfactants, semi-polar nonionic surfactants and mixtures thereof.

The surfactant is typically present at a level of from about 0.1% to about 60%, from about 1% to about 50% or even from about 5% to about 40% by weight of the subject composition.

Chelating Agents - The compositions herein may contain a chelating agent. Suitable chelating agents include copper, iron and/or manganese chelating agents and mixtures thereof. When a chelating agent is used, the composition may comprise from about 0.1% to about 15% or even from about 3.0% to about 10% chelating agent by weight of the subject composition.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof.

When present in a subject composition, the dye transfer inhibiting agents may be present at levels from about 0.0001% to about 10%, from about 0.01% to about 5% or even from about 0.01% to about 3% by weight of the composition.

Dispersants - The compositions of the present invention can also contain dispersants. Suitable water-soluble organic materials include the homo- or co-polymeric acids or their salts, in which the polycarboxylic acid comprises at least two carboxyl radicals separated from each other by not more than two carbon atoms.

Perfumes - The dispersed phase may comprise a perfume that may include materials selected from the group consisting of perfumes such as 3-(4-*t*-butylphenyl)-2-methyl propanal, 3-(4-*t*-butylphenyl)-propanal, 3-(4-isopropylphenyl)-2-methylpropanal, 3-(3,4-methylenedioxyphenyl)-2-methylpropanal, and 2,6-dimethyl-5-heptenal, $\alpha$-damascone, $\beta$-damascone, $\delta$-damascone, $\beta$-damascenone, 6,7-dihydro-1,1,2,3,3-pentamethyl-4(5H)-indanone, methyl-7,3-dihydro-2H-1,5-benzodioxepine-3-one, 2-[2-(4-methyl-3-cyclohexenyl-1-yl)propyl]cyclopentan-2-one, 2-sec-butylcyclohexanone, and $\beta$-dihydro ionone, linalool, ethyllinalool, tetrahydrolinalool, and dihydromyrcenol.

Perfume Delivery Technologies - The compositions of the present invention may comprise one or more perfume delivery technologies that stabilize and enhance the deposition and release of perfume ingredients from treated substrate. Such perfume delivery technologies can also be used to increase the longevity of perfume release from the treated substrate. Perfume delivery technologies, methods of making certain perfume delivery technologies and the uses of such perfume delivery technologies are disclosed in US 2007/0275866 A1.

In one aspect, the compositions of the present invention may comprise from about 0.001% to about 20%, or from about 0.01% to about 10%, or from about 0.05% to about 5%, or even from about 0.1% to about 0.5% by weight of the perfume delivery technology. In one aspect, said perfume delivery technologies may be selected from the group consisting of: perfume microcapsules, pro-perfumes, polymer particles, functionalized silicones, polymer assisted delivery, molecule assisted delivery, fiber assisted delivery, amine assisted delivery, cyclodextrins, starch encapsulated accord, zeolite and inorganic carrier, and mixtures thereof:

Said perfume delivery technology may comprise microcapsules formed by at least partially surrounding a benefit agent with a wall material. The microcapsule wall material may comprise: melamine, polyacrylamide, silicones, silica, polystyrene, polyurea, polyurethanes, polyacrylate based materials, gelatin, styrene malic anhydride, polyamides, and mixtures thereof. Said melamine wall material may comprise melamine crosslinked with formaldehyde, melamine-dimethoxyethanol crosslinked with formaldehyde, and mixtures thereof. Said polystyrene wall material may comprise polyestyrene cross-linked with divinylbenzene. Said polyurea wall material may comprise urea crosslinked with formaldehyde, urea crosslinked with gluteraldehyde, and mixtures thereof. Said polyacrylate based materials may comprise polyacrylate formed from methylmethacrylate/dimethylaminomethyl methacrylate, polyacrylate formed from amine acrylate and/or methacrylate and strong acid, polyacrylate formed from carboxylic acid acrylate and/or methacrylate monomer and strong base, polyacrylate formed from an amine acrylate and/or methacrylate monomer and a carboxylic acid acrylate and/or carboxylic acid methacrylate monomer, and mixtures thereof. The perfume microcapsule may be coated with a deposition aid, a cationic polymer, a non-ionic polymer, an anionic polymer, or mixtures thereof. Suitable polymers may be selected from the group consisting of: polyvinylformaldehyde, partially hydroxylated polyvinylformaldehyde, polyvinylamine, polyethyleneimine, ethoxylated polyethyleneimine, polyvinylalcohol, polyacrylates, and combinations thereof. The microcapsule may be a perfume microcapsule. One or more types of microcapsules, for example two microcapsules types having different benefit agents may be used.

Said perfume delivery technology may comprise an amine reaction product (ARP) or a thio reaction product. One may also use "reactive" polymeric amines and or polymeric thiols in which the amine and/or thiol functionality is pre-reacted with one or more PRMs to form a reaction product. Typically, the reactive amines are primary and/or secondary amines, and may be part of a polymer or a monomer (non-polymer). Such ARPs may also be mixed with additional PRMs to provide benefits of polymer-assisted delivery and/or amine-assisted delivery. A material that contains a heteroatom other than nitrogen and/or sulfur, for example oxygen, phosphorus or selenium, may be used as an alternative to amine compounds. The alternative compounds can be used in combination with amine compounds. A single molecule may comprise an amine moiety and one or more of the alternative heteroatom moieties, for example, thiols, phosphines and selenols. The benefit may include improved delivery of perfume as well as controlled perfume release. Suitable ARPs as well as methods of making same can be found in USPA 2005/0003980 A1 and USP 6,413,920 B1.

## Fabric Softening Actives

The compositions of the present invention may comprise a fabric softening active ("FSA"). Suitable fabric softening actives, include, but are not limited to, materials selected from the group consisting of quaternary ammonium compounds (quat), quaternary ammonium ester compounds, amines, fatty esters, sucrose esters, silicones, dispersible polyolefins, clays, polysaccharides, fatty acids, softening oils, polymer latexes and mixtures thereof.

Non-limiting examples of water insoluble fabric care benefit agents include dispersible polyethylene and polymer latexes.

The quaternary ammonium ester softening active

The quaternary ammonium ester softening active may be present at a level of from 3.0% to 25.0%, more preferably from 4.0% to 18%, even more preferably from 4.5% to 15% by weight of the composition. The level of quaternary ammonium ester softening active may depend of the desired concentration of total softening active in the composition (diluted or concentrated composition) and of the presence or not of other softening active. While higher FSA levels improve the softness benefits, the risk on instabilities is typically higher in fabric softener compositions with higher FSA levels.

Suitable quaternary ammonium ester softening actives include but are not limited to, materials selected from the group consisting of monoester quats, diester quats, triester quats and mixtures thereof. Preferably, the level of monoester quat is from 2.0% to 40.0%, the level of diester quat is from 40.0% to 98.0%, the level of triester quat is from 0.0% to 25.0% by weight of total quaternary ammonium ester softening active.

Said quaternary ammonium ester softening active may comprise compounds of the following formula:

$$\{R^2_{(4-m)} - N+ - [X - Y - R^1]_m\} \ A^-$$

wherein:

m is 1, 2 or 3 with proviso that the value of each m is identical;
each $R^1$ is independently hydrocarbyl, or branched hydrocarbyl group, preferably $R^1$ is linear, more preferably $R^1$ is partially unsaturated linear alkyl chain;
each $R^2$ is independently a $C_1$-$C_3$ alkyl or hydroxyalkyl group, preferably $R^2$ is selected from methyl, ethyl, propyl, hydroxyethyl, 2-hydroxypropyl, 1-methyl-2-hydroxyethyl, poly($C_{2-3}$ alkoxy), polyethoxy, benzyl;
each X is independently -$(CH_2)$n-, -$CH_2$-$CH(CH_3)$- or -CH-$(CH_3)$-$CH_2$- and
each n is independently 1, 2, 3 or 4, preferably each n is 2;
each Y is independently -O-(O)C- or -C(O)-O-;
$A^-$ is independently selected from the group consisting of chloride, methyl sulfate, and ethyl sulfate, preferably $A^-$ is selected from the group consisting of chloride and methyl sulfate;

with the proviso that when Y is -O-(O)C-, the sum of carbons in each $R^1$ is from 13 to 21, preferably from 13 to 19. Preferably, X is -$CH_2$-$CH(CH_3)$- or -CH-$(CH_3)$-$CH_2$- to improve the hydrolytic stability of the quaternary ammonium ester softening active, and hence further improve the stability of the fabric softener composition.

In preferred liquid fabric softener compositions the iodine value of the parent fatty acid from which the quaternary ammonium fabric softening active is formed is from 0 to 100, more preferably from 10 to 60, even more preferably from 15 to 45.

Examples of suitable quaternary ammonium ester softening actives are commercially available from KAO Chemicals under the trade name Tetranyl AT-1 and Tetranyl AT-7590, from Evonik under the tradename Rewoquat WE16 DPG, Rewoquat WE18, Rewoquat WE20, Rewoquat WE28, and Rewoquat 38 DPG, from Stepan under the tradename Stepantex GA90, Stepantex VR90, Stepantex VK90, Stepantex VA90, Stepantex DC90, Stepantex VL90A.

These types of agents and general methods of making them are disclosed in U.S.P.N. 4,137,180.

Silicone

The compositions of the present invention may comprise a silicone. Suitable levels of silicone may comprise from about 0.1% to about 70%, alternatively from about 0.3% to about 40%, alternatively from about 0.5% to about 30%, alternatively from about 1% to about 20% by weight of the composition. Useful silicones can be any silicone comprising compound. In one embodiment, the silicone polymer is selected from the group consisting of cyclic silicones, polydimethylsiloxanes, aminosilicones, cationic silicones, silicone polyethers, silicone resins, silicone urethanes, and mixtures thereof. The silicone is a polydialkylsilicone, alternatively a polydimethyl silicone (polydimethyl siloxane or "PDMS"), or a derivative thereof. The silicone is chosen from an aminofunctional silicone, amino-polyether silicone, alkyloxylated silicone, cationic silicone, ethoxylated silicone, propoxylated silicone, ethoxylated/propoxylated silicone, quaternary silicone, or combinations thereof.

## TEST METHODS

Malodor reduction materials may be separated from mixtures, including but not limited to finished products such as consumer products and identified, by analytical methods that include GC-MS and/or NMR.

MORV and Universal MORV calculation

1.) Search Chemical Abstracts using the name and/or the CAS Registry number for the material of interest and use the structure provided. If there are multiple isomers for a given material, use the structure provided by Chemical Abstracts or use the isomer that is most prevalent in the material. Input structure of material into winMolconn (Hall Associates, version 1.1.2.1).

2.) Input values from 1.) above into the following four equations:

a) MORV =-8.5096 + 2.8597x(dxp9) + 1.1253×(knotpv) - 0.34484×(e1C2O2) - 0.00046231×(idw) + 3.3509×(idcbar) + 0.11158×(n2pag22)

b) MORV = -5.2917 + 2.1741×(dxvp5) - 2.6595×(dxvp8) + 0.45297×(e1C2C2d) - 0.6202×(c1C2O2) + 1.3542×(CdCH2) + 0.68105×(CaasC) + 1.7129×(idcbar)

c) MORV =-0.0035 + 0.8028×(SHCsatu) + 2.1673×(xvp7) - 1.3507×(c1C1C3d) + 0.61496×(c1C1O2) + 0.00403×(idc) - 0.23286×(nd2).

d) MORV = -0.9926 - 0.03882×(SdO) + 0.1869×(Ssp3OH) + 2.1847×(xp7) + 0.34344×(e1C3O2) - 0.45767×(c1C2C3) + 0.7684×(CKetone)

Equation a) is for the malodor trans-3-methyl-2-hexenoic acid (carboxylic acid based malodors)
Equation b) is for the malodor trimethylamine (amine based malodors)
Equation c) is for the malodor 3-mercapto-3-methylhexan-1-ol (thiol based malodors)
Equation d) is for the malodor skatole (indole based malodors)

3.) For purpose of the present application, a material's MORV is the highest MORV value from equations 2.)a) through 2.)d).

4.) If all MORV values from equations 2.)a) through 2.)d) above are greater than 0.5, the subject material has a Universal MORV.

**EXAMPLE 1: Malodor reduction composition comprising entirely non-perfume materials**

| Ingredient | CAS # | MRC A Percent | MRC B Percent | MRC C Percent | MRC D Percent | MRC E Percent |
|---|---|---|---|---|---|---|
| (E)-1,1,4,4-tetramethoxybut-2-ene | 6068-62-8 | 38.500 | 0.00 | 48.00 | 48.00 | 0.00 |
| 1,2,3,4,5,6-hexamethylbenzene | 87-85-4 | 19.500 | 0.00 | 0.00 | 23.50 | 23.50 |
| dimethyl (4R,5R)-2,2-dimethyl-1,3-dioxolane-4,5-dicarboxylate | 37031-29-1 | 4.000 | 10.00 | 5.00 | 5.00 | 6.00 |
| 3,5-Dimethyladamantane-1-carboxylic acid | 14670-94-1 | 19.000 | 45.00 | 23.50 | 23.50 | 23.50 |
| (1R,6S)-6-(methoxycarbonyl)cyclohex-3-ene-1-carboxylic acid | 88335-93-7 | 19.000 | 45.00 | 23.50 | 0.00 | 23.50 |
| dimethyl (1R,2S)-cyclohex-4-ene-1,2-dicarboxylate | 4841-84-3 | 0.000 | 0.00 | 0.00 | 0.00 | 23.50 |
| Total | | 100.00 | 100.00 | 100.00 | 100.00 | 100.00 |

EXAMPLE 2: Malodor reduction composition comprising non-perfume and perfume materials functioning as malodor reduction materials such as those disclosed in patent application serial number 14/864,927, Frankenbach et al.

| Ingredient | CAS# | MRC F Percent | MRC G Percent | MRC H Percent | MRC J Percent | MRC K Percent |
|---|---|---|---|---|---|---|
| (E)-1,1,4,4-tetramethoxybut-2-ene | 6068-62-8 | 38.500 | 0.00 | 48.00 | 48.00 | 20.00 |
| 1,2,3,4,5,6-hexamethylbenzene | 87-85-4 | 19.500 | 0.00 | 0.00 | 23.50 | 14.00 |
| dimethyl (4R,5R)-2,2-dimethyl-1,3-dioxolane-4,5-dicarboxylate | 37031-29-1 | 4.000 | 10.00 | 0 | 5.00 | 9.00 |
| 3,5-Dimethyladamantane-1-carboxylic acid | 14670-94-1 | 9.00 | 45.00 | 23.50 | 3.00 | 23.50 |
| (1R,6S)-6-(methoxycarbonyl)cyclohex-3-ene-1-carboxylic acid | 88335-93-7 | 19.000 | 0.00 | 23.50 | 0.00 | 0.00 |

(continued)

| Ingredient | CAS# | MRC F Percent | MRC G Percent | MRC H Percent | MRC J Percent | MRC K Percent |
|---|---|---|---|---|---|---|
| dimethyl (1R,2S)-cyclohex-4-ene-1,2-dicarboxylate | 4841-84-3 | 0.000 | 0.00 | 0.00 | 0.00 | 0.00 |
| 1,3,4,6,7,8alpha-hexahydro-1,1,5,5-tetramethyl-2H-2,4alpha-methanophthalen-8(5H)-one | 23787-90-8 | 10.000 | 0.000 | 0.000 | 7.00 | 14.00 |
| (E)-3,7-dimethylocta-2,6-dien-1-yl palmitate | 3681-73-0 | 0.000 | 45.00 | 0.000 | 0.000 | 0.000 |
| 3-(6,6-dimethylbicyclo[3.1.1]hept-2-en-2-yl)-2,2-dimethylpropanal | 33885-52-8 | 0.000 | 0.000 | 5.00 | 0.000 | 0.000 |
| 1-((2-(tert-butyl)cyclohexyl)oxy)butan-2-ol | 139504-68-0 | 0.000 | 0.000 | 0.000 | 13.50 | 10.00 |
| (R,Z)-1-(2,6,6-trimethylcyclohex-2-en-1-yl)pent-1-en-3-one | 127-42-4 | 0.000 | 0.000 | 0. 000 | 0.000 | 9.50 |
| Total | | 100.00 | 100.00 | 100.00 | 100.00 | 100.00 |

The term "air refresher" or "air freshener", as used herein, refer to any suitable composition that reduces odors in air, and/or reduces the impression of odors in the air by masking, layering or including malodor counteractant perfume raw materials into the composition.

Example 2.1-2.3: Energized Air Refresher

An energized air refresher composition is prepared with malodor reduction composition, according to the compositions shown in Example 1.

| | Example 2.1 | Example 2.2 | Example 2.3 |
|---|---|---|---|
| Perfume composition | 40-60 | 50-60 | 30-50 |
| Malodor reducing composition | 10-20 | 15-30 | 10-25 |
| Functional Perfume Component | 30-50 | 10-30 | 25-60 |

Use of Functional Perfume Components at the recited levels may help modulate the evaporation profile of an entire perfume composition to provide perfume character consistency over the intended usage period.

Suitable FPCs may be highly volatile, low boiling, perfume ingredients. Non-limiting, suitable FPCs include iso-nonyl acetate, dihydro myrcenol (3 -methylene-7-methyloctan-7-ol), linalool (3-hydroxy-3,7-dimethyl-1,6 octadiene), geraniol (3,7 dimethyl-2,6-octadien-1-ol), d-limonene,1 -methyl-4-isopropenyl-1-cyclohexene, benzyl acetate, isopropyl mystristate, and combinations thereof.

Functional Perfume Component Examples 2.1-2.3

| | Example A1 | Example A2 | Example A3 |
|---|---|---|---|
| Benzyl alcohol | 10-50 | 0-50 | 0-20 |
| Iso nonyl acetate | 1-20 | 1-10 | 1-10 |
| Dipropylene glycol methyl ether | 40-70 | 5-65 | 2-50 |

An energized air freshening device is filled with the composition above. The resulting air freshener is effective at reducing malodor.

Examples 3.1-3.3 - Non-Energized Air Freshener

A non-energized air refresher composition is prepared with malodor reduction composition, according to the compositions shown in Example 1.

|  | Example 3.1 | Example 3.2 | Example 3.3 |
|---|---|---|---|
| Perfume composition | 20-30 | 20-50 | 10-30 |
| Malodor reducing composition | 10-20 | 10-20 | 20-50 |
| Functional Perfume components | 0-70 | 40-70 | 20-70 |

| Functional Perfume components | Example 1 | Example 2 | Example 3 |
|---|---|---|---|
| 3a,4,5,6,7,7a-hexahydro-1H-4,7-methanoinden-1-yl propanoate | 15-25 | 20-25 | 0-20 |
| Iso nonyl acetate | 30-75 | 15-75 | 1-10 |
| 2-isobutyl-4-hydroxy-4-methyltetrahydropyran | 20-30 | 5-65 | 2-50 |

A non-energized air freshener is filled with the example composition above. The resulting air freshener is effective at reducing malodor.

Examples 4.1-4.2 - Fabric and Air Refresher Composition

A fabric refresher composition is prepared with malodor reduction composition, according to the compositions shown in Example 1.

| Ingredient | Example 4.1 | Example 4.2 | Example 4.3 (ranges) |
|---|---|---|---|
| Deionized Water | Balance | Balance | Balance |
| Ethanol | 3.0 | 3.0 | 1-5.0% |
| Lupasol HF+ | 0.0650 | 0.0650 | 0-0.1% |
| Diethylene Glycol | 0.175 | 0.175 | 0-0.2% |
| Silwet L-7600 | 0.1 | 0.100 | 0-0.2 |
| Maleic Acid and/or Citric Acid | 0.05 | 0.05 | 0-0.2 |
| Koralone B-119 | 0.015 | 0.015 | 0-0.1 |
| Hydroxypropyl β-cyclodextrin | 0.630 | 0.630 | 0-2.0% |
| Sodium Hydroxide | 0.003 | 0.003 | 0.001-0.01 |
| Fragrance | 0 | 0.4% | 0-1.0% |
| Malodor Reduction Composition (MRC A, B, C, D, E, F, G, H, J, K) | 0.03% | 0.04% | 0-0.1% |
| Total | 100.000 | 100.000 | 100.000 |

Lupasol HF        Polyethyleneimine (available from BASF)
Silwet L-7600     Organosilicone (available from BASF)
Koralone B-119    19% active aqueous solution of 1,2 Benzisothiazolin-3-one (BIT) in dipropylene glycol and water (available from Dow Chemical)

The resulting Fabric fresher composition is effective at reducing or preventing malodor when sprayed on fabrics, such as articles of clothing or upholstery.

Example 5.1-5.2 - Aerosol Air Freshener Composition

An aerosol air freshener composition is prepared with malodor reduction composition, according to the compositions shown in Example 1.

| Ingredient | Example 5.1 | Example 5.2 |
|---|---|---|
| Water Purified USP | Balance | 92.370 |
| Ethyl Alcohol | 5.000 | 5.000 |
| Acid Polyacrylic | 0.185 | - |
| Sodium Citrate,Dihydrate | - | 0.100 |
| Acid Citric Solution 50% | - | 0.100 |
| Sodium Dicoctyl Sulfosuccinate | 0-0.200 | - |
| Silwet L-7600 | 0 - 0.2 | 0.200 |
| Hydrogenated Castor Oil PEG 60 | 1.400 | 1.400 |
| Fragrance | 0- 1.5% | 0 - 1.5% |
| Malodor Reduction Composition (MRC A, B, C, D, E, F, G, H, J, or K) | 1.0 | 0 - 1.50% |
| Hydroxypropyl BetaCyclodextrine | 0.380 | 0.380 |
| Koralone B-119 | 0.050 | 0.050 |
| Sodium Hydroxide 20% | 0 - 0.3 | - |
| Total | 100 | 100 |

| | |
|---|---|
| Silwet L-7600 | Organosilicone (available from BASF) |
| Koralone B-119 | 19% active aqueous solution of 1,2 Benzisothiazolin-3-one (BIT) in dipropylene glycol and water (available from Dow Chemical) |

A resulting aerosol air freshener is effective at reducing malodor in the air when sprayed. In addition, the aerosol can be sprayed on fabrics to reduce or prevent malodor.

Examples 6.1-6.4 - Heavy Duty Liquid Composition

A HDL-Heavy Duty Liquid composition is prepared with malodor reduction composition, according to the compositions shown in Example 1.

| | Example 6.1 | Example 6.2 | Example 6.3 | Example 6.4 |
|---|---|---|---|---|
| Ingredient | WT% Active | WT% Active | WT% Active | WT% Active |
| $AE_{1.8}S$ | 16.3 | 16.3 | 12 | 8 |
| $C_{11.8}$ linear alkyl benzene sulfonic acid | 2.8 | 2.8 | 8 | -- |
| HSAS | 13.6 | 13.6 | 0 | 22 |
| C24 alcohol. EO9 | 2.2 | 2.2 | 1 | 1.8 |
| Citric Acid | 0.9 | 0.9 | 2 | 1.7 |
| Lactic Acid | --- | 5.8 | --- | --- |
| $C_{12}$-$C_{18}$ Fatty Acid | 2.3 | 1.3 | 0.8 | 3.0 |

(continued)

| Ingredient | Example 6.1 WT% Active | Example 6.2 WT% Active | Example 6.3 WT% Active | Example 6.4 WT% Active |
|---|---|---|---|---|
| Protease (55.3 mg/g) | 1.7 | 1.7 | 1.7 | 1.7 |
| Amylase (25.4mg/g) | 0.7 | 0.7 | 0.7 | 0.7 |
| Borax | 3.6 | 3.6 | 3.6 | 3.6 |
| Calcium Formate | 0.2 | 0.2 | 0.2 | 0.2 |
| Polyethyleneimine 600, EO20 | 1.6 | 1.6 | --- | 1.6 |
| Polyethyleneimine 600, EO24, PO16 | 1.6 | --- | 2.0 | 1.6 |
| DTPA | 0.3 | 0.3 | 0.3 | 0.3 |
| Tiron® | 0.8 | 0.8 | 0.8 | 0.8 |
| Optical Brightener [7] | 0.3 | 0.3 | 0.3 | 0.3 |
| NaOH | 4.0 | 9.3 | 4.0 | 9.3 |
| Na Cumene Sulfonate | 1.1 | 1.1 | 1.1 | 1.1 |
| Na Formate | 0.2 | 0.2 | 0.2 | 0.2 |
| Aesthetics Dye | 0.03 - 1.0 | 0.03 - 1.0 | 0.03 - 1.0 | 0.03 - 1.0 |
| Perfume | 0.5 - 3.0 | 0.5 - 3.0 | 0.5 - 3.0 | 0.5 - 3.0 |
| Malodor Reduction Composition (MRC A, B, C, D, E, F, G, H, J, or K) | 0.15-1.0 | 0.15-1.0 | 0.15-1.0 | 0.15-1.0 |
| Water and Solvent | Balance | Balance | Balance | Balance |
| pH | 5.0 | 8.0 | 5.0 | 8.0 |

HSAS     secondary alkyl sulfate, acid form
DTPA     diethylene triamine pentaacetic acid (DTPA)
Tiron     4,5-Dihydroxy-1,3-benzenedisulfonic acid disodium salt monohydrate

The resulting heavy duty liquid product when used to wash articles of clothing is effective at reducing malodor on the washed clothing.

Examples 7.1-7.3 - Heavy Duty Liquid Composition

Other examples of HDL-Heavy Duty Liquid composition prepared with malodor reduction composition, according to the compositions shown in Example 1.

| Ingredient | Example 7.1 WT% Active | Example 7.2 WT% Active | Example 7.33 WT% Active |
|---|---|---|---|
| $AE_{1.8}S$ | 14% | 8% | 11% |
| Amine Oxide | 2% | --- | --- |
| $C_{11.8}$ linear alkyl benzene sulfonic acid | 10% | --- | --- |
| HSAS | --- | 20% | 15 |
| HF LAS | --- | --- | --- |
| C24 alcohol. EO9 | 1% | 2% | 1.2% |
| Citric Acid | 3% | 1.5% | 0.9% |

(continued)

| Ingredient | Example 7.1 WT% Active | Example 7.2 WT% Active | Example 7.33 WT% Active |
|---|---|---|---|
| $C_{12}$-$C_{18}$ Fatty Acid | 2% | 1% | 1.5% |
| Protease (55.3 mg/g) | 0.013 | 0.8 | 0.013 |
| Amylase (25.4mg/g) | 0.02 | 0.7 | 0.02 |
| Borax | 2 - 8.0 | 2 - 8.0 | 2 - 8.0 |
| Calcium Formate | 0.2 | 0.2 | 0.2 |
| Carezyme 5.0L | 0.01 - 0.5 | --- | 0.01 - 0.5 |
| Polyethyleneimine 600, EO20 | 0.5 - 3.0 | 0.5 - 3.0 | 0.5 - 3.0 |
| Polyethyleneimine 600, EO24, PO16 | 0.5 - 3.0 | --- | 0.5 - 3.0 |
| DTPA | 0.1 - 1.0 | 0.1 - 1.0 | 0.1 - 1.0 |
| Tiron® | 0.1 - 1.5 | 0.1 - 1.5 | 0.1 - 1.5 |
| Optical Brightener | 0.1 - 1.0 | 0.1 - 1.0 | 0.1 - 1.0 |
| MEA- Hydrogenated Castor Oil | 0.5 - 3.0 | --- | |
| NaOH | 0.1 - 1.0 | 0.1-1.0 | |
| Na Cumene Sulfonate | 1.1 | --- | |
| Na Formate | 0.2 | 0.2 | |
| Dye | 0.1 | 0.1 | |
| Malodor Reduction Composition (MRC A, B, C, D, E, F, G, H, J, or K) | 0.15-1.0 | 0.15-1.0 | 0.15-1.0 |
| Perfume | 0.9 | 0.5 - 3 | |
| Water and Solvent | Balance | Balance | Balance |

HSAS    secondary alkyl sulfate, acid form
DTPA    diethylene triamine pentaacetic acid (DTPA)
Tiron    4,5-Dihydroxy-1,3-benzenedisulfonic acid disodium salt monohydrate

The resulting heavy duty liquid product when used to wash articles of clothing is effective at reducing malodor on the washed clothing.

Examples 8.1-8.6 - Soluble Uni-dose Heavy Duty Liquid Composition

An example for a Soluble Uni-dose heavy duty liquid composition prepared with malodor reduction composition, according to the compositions shown in Example 1.

| | 8.1 | 8.2 | 8.3 | 8.4 | 8.5 | 3 compartments pouched product 8.6 | | |
|---|---|---|---|---|---|---|---|---|
| Form | liquid | liquid | liquid | liquid | gel | liq | liq | liq |
| Compartment # | 1 | 1 | 1 | 1 | 1 | 1 | 2 | 3 |
| Dosage (g) | 36.0 | 38.0 | 32.0 | 36.0 | 40.0 | 34.0 | 25 | 35 |
| Alkylbenzene sulfonic acid | 14.5 | 13.8 | 16.0 | 14.5 | 13.5 | 14.5 | 20.0 | |
| $C_{12-14}$ alkyl ethoxy 3 sulfate | 8.5 | 16.4 | 10.0 | 8.5 | 15.0 | 8.5 | | |
| $C_{12-13}$ alkyl 3-ethoxylate | | | | 13.0 | | | | |

(continued)

| | 8.1 | 8.2 | 8.3 | 8.4 | 8.5 | 3 compartments pouched product 8.6 | | |
|---|---|---|---|---|---|---|---|---|
| Form | liquid | liquid | liquid | liquid | gel | liq | liq | liq |
| $C_{12-14}$ alkyl 7-ethoxylate | 12.5 | 9.0 | 14.0 | | 4.0 | 12.5 | 17.0 | |
| C12-18 Fatty acid | 14.5 | 8.5 | 16.0 | 15.0 | 7.2 | 14.5 | 13.0 | |
| Citric acid | | | | 2.0 | 4.1 | | | |
| Enzymes | 0-3 | 0-3 | 0-3 | | 0-3 | 0-3 | 0-3 | |
| PAP granule [1] | | | | | | | | 50.0 |
| Ethoxysulfated Hexamethylene Diamine Dimethyl Quat | | 3.0 | | | | | 2.2 | |
| Ethoxylated Polyethylenimine | 4.0 | 1.0 | | 4.0 | 3.0 | 2.0 | | |
| Hydroxyethane diphosphonic acid | 1.0 | 1.0 | | | 1.6 | 0.6 | 0.6 | |
| Ethylene diamine tetra (methylene phosphonic) acid | | | | 1.0 | | | | |
| Brightener | 0.2 | 0.2 | 0.3 | 0.3 | 0.2 | 0.2 | 0.2 | |
| Polydimethyl Siloxane | | | 3.0 | | | | | |
| Hueing dye [2] | | | | | | | 0.05 | |
| Perfume | 1.5-2.0 | 1.5-2.0 | 1.5-2.0 | 1.5-2.0 | 1.5-2.0 | 1.5-2.0 | | |
| Malodor Reduction Composition (MRC A, B, C, D, E, F, G, H, J, orK) | 0.3-0.7 | 0.3-0.7 | 0.3-0.7 | 0.3-0.7 | 0.3-0.7 | 0.3-0.7 | | |
| Water and minors | To 100% | | | | | | | |
| Buffers (sodium carbonate, monoethanolam ine) | To pH 8.0 | | | | | | | |
| Solvents (1,2 propanediol, ethanol), Sulfate | To 100% | | | | | | | |
| 1 PAP granule 2 Hueing dye | | | | | | | | |

The resulting Unidose pouch product when used to wash articles of clothing is effective at reducing malodor on the washed clothing.

Examples 9.1-9.5 - Fabric Enhancer Solid Particle or Bead Composition

An example of a Fabric Enhancer bead composition prepared with malodor reduction composition, according to the compositions shown in Example 1.

| | Example 9.1 | Example 9.2 | Example 9.3 | Example 9.4 | Example 9.5 |
|---|---|---|---|---|---|
| Ingredients | Active Wt% | Active Wt% | Active Wt% | Active Wt% | Active Wt% |
| PEG 8000 | 86.21 | 83.255 | 83.255 | 86.21 | 82.49 |
| Free (Neat) Perfume | 4.41 | 5.88 | 4.41 | 4.25 | 5.88 |
| MRC | 0.75-1.5 | 0.75-1.5 | 0.75-1.5 | 0.75-1.5 | 0.75-1.5 |
| Perfume Micro Capsule (PMC) | 5.88 | 8.22 | 8.88 | 5.88 | 8.70 |

(continued)

| | Example 9.1 | Example 9.2 | Example 9.3 | Example 9.4 | Example 9.5 |
|---|---|---|---|---|---|
| Ingredients | Active Wt% | Active Wt% | Active Wt% | Active Wt% | Active Wt% |
| Encapsulated Perfume | 1.88 | 1.88 | 1.88 | 1.88 | 1.88 |
| Malodor Reducing Composition | 1.47 | --- | 1.47 | 1.63 | --- |
| Encapsulated Malodor Reducing Composition | --- | 0.75 | 0.75 | --- | 0.9 |
| Aesthetics Dye | 0.015 | 0.015 | 0.015 | 0.015 | 0.015 |
| 1. PMC is a friable PMC with a urea-formaldehyde shell from Appvion-Encapsys. About 50% water by weight of the PMC (including encapsulated perfume) is assumed. 2. Encapsulated perfume (within PMC) assumes about 32% active | | | | | |

The resulting Fabric enhancing bead product when added to an automatic washing machine is effective at reducing malodor on the clothing.

Examples 10.1-10.4 - Dryer Added Fabric Softener Sheet Composition

An example of a dryer added fabric softener sheet composition prepared with malodor reduction composition, according to the compositions shown in Example 1.

| | Example 10.1 | Example 10.2 | Example 10.3 | Example 10.4 |
|---|---|---|---|---|
| Ingredient | Wt% Active | Wt% Active | Wt% Active | Wt% Active |
| DEQA[1] | --- | 50 | --- | --- |
| DEQA[2] | 30 | --- | --- | 30 |
| DTDMAMS[3] | --- | --- | 50 | --- |
| 7018FA[4] | --- | --- | 50 | --- |
| TS-20[5] | 15 | --- | --- | 15 |
| SMS[6] | 15 | --- | --- | 15 |
| SDASA[7] | 19 | 25 | --- | 19 |
| TPED[8] | --- | 3 | --- | --- |
| Complex[9] | 16.5 | 16.5 | --- | 8.0 |
| Clay[10] | 3 | 4 | --- | 3 |
| Free (Neat) Perfume | 1.5 | 1.5 | --- | 1.5 |
| Free (Neat) malodor reducing composition | 0.5 | 0.5 | 0.5 | --- |
| Encapsulated Perfume/malodor reducing composition [11] | --- | --- | --- | 1.56 |
| Encapsulated Perfume[12] | --- | 0.4 | --- | --- |
| Encapsulated malodor reducing composition | --- | --- | --- | 0.5 |

(continued)

| | Example 10.1 | Example 10.2 | Example 10.3 | Example 10.4 |
|---|---|---|---|---|
| Ingredient | Wt% Active | Wt% Active | Wt% Active | Wt% Active |
| Active Weight (g/sheet) | 2.4 | 2.4 | 1.9 | 2.4 |

(1) DEQA[1]: Di(soft tallowoyloxyethyl)dimethylammonium methyl sulfate with 25%> 7018 FA, as described below, as solvent
(2) DEQA[2]: Di(soft tallowoyloxyethyl)hydroxyethylmethylammoniun methyl sulfate with 18%» partially hydrogenated tallow fatty acid solvent
(3) DTDMAMS: Di(hydrogenated tallowalkyl)dimethylammonium methyl sulfate
(4) 7018FA: 70:30 Stearic Acid:Palmitic Acid (IV=0) Industrene 7018 sold by Witco
(5) TS-20: Polyoxyethylene-20 Sorbitan Tristearate (Glycosperse TS-20, sold by Lonza
(6) SMS: Sorbitan Mono Stearate
(7) SDASA: 1 :2 ratio of stearyl dimethyl amine: triple pressed stearic acid
(8) TPED: N,N,N',N'-Tetrakis(2-hydroxypropyl)ethylenediamine (Quadrol, sold by BASF)
(9) Complex: Beta-Cyclodextrin/Perfume Complex
(10) Clay: Calcium Bentonite Clay (Bentonite L sold by Southern Clay Products Free (Neat) Perfume
(11) PMC is a friable PMC with a urea-formaldehyde shell from Appleton. About 50% water by weight of the PMC (including encapsulated perfume and/ or blocker) is assumed.
(12) Encapsulated perfume and encapsulated malodor reducing composition (within PMC) assumes about 32% active

The compositions of Example 10 are mixed homogeneously and impregnated onto a non- woven polyester sheet having dimensions of about 6% in x 12" (about 17.1 cm x 30.5 cm) and weighing about 1 gram.
The resulting dryer added fabric softener sheet product when added to an automatic dryer is effective at reducing malodor on the clothing.

Examples 11.1-11.4 - Liquid Fabric Enhancer Composition

An example of a Liquid Fabric Enhancer composition prepared with malodor reduction composition, according to the compositions shown in Example 1.

| | Example 11.1 | Example 11.2 | Example 11.3 | Example 11.4 |
|---|---|---|---|---|
| Ingredient | Wt% Active | Wt% Active | Wt% Active | Wt% Active |
| FSA[1] | 12 | 21 | 18 | 14 |
| Low MW alcohol | 1.95 | 3.0 | 3.0 | 2.28 |
| Structurant[2,3] | 1.25[e] | - | 0.2[f] | - |
| Free (Neat) Perfume | 1.50 | 1.8 | 2.0 | 1.50 |
| Free (Neat) malodor reducing composition | 0.3 | 0.7 | - | - |
| Encapsulated Perfume[4] | - | 0.6 | - | 0.6 |
| Encapsulated malodor reducing composition [4] | - | - | 0.6 | 0.6 |
| Perfume/ malodor reducing composition encapsulated[5] | - | 1.85 | 1.85 | 3.7 |
| Calcium Chloride | 0.10 | 0.12 | 0.1 | 0.45 |
| DTPA[6] | 0.005 | 0.005 | 0.005 | 0.005 |
| Preservative (ppm)[7] | 5 | 5 | 5 | 5 |
| Antifoam[8] | 0.015 | 0.15 | 0.11 | 0.011 |
| Polyethylene imines[9] | 0.15 | 0.05 | - | 0.1 |
| Delivery enhancing | 0.1 | 0.1 | 0.2 | 0.05 |
| PDMS emulsion[10] | - | 0.5 | 1 | 2.0 |

(continued)

| Ingredient | Example 11.1 Wt% Active | Example 11.2 Wt% Active | Example 11.3 Wt% Active | Example 11.4 Wt% Active |
|---|---|---|---|---|
| Dispersant[11] | - | - | 0.5 | 0.2 |
| Organosiloxane | 5 | - | - | - |
| Front-end Stability Aid[12,13] | 0.06[11] | 0.63[12] | 0.36[11] | 0.14[12] |
| Dye (parts per million ppm) | 40 | 11 | 30 | 40 |
| Ammonium Chloride | | | | 0.10 |
| Hydrochloric Acid | 0.010 | 0.01 | 0.10 | 0.010 |
| Deionized Water | Balance | Balance | Balance | Balance |

(1) N,N-di(tallowoyloxyethyl)-N-dimethylammonium chloride.
(2) Cationic high amylose maize starch-available from National Starch under the trade name HYLON VII ®.
(3) Cationic polymer available from BASF ® under the name Rheovis ® CDE.
(4) Encapsulated perfume and encapsulated malodor reducing composition (within PMC) assumes about 32% active
(5) PMC is a friable PMC with a urea-formaldehyde shell from Appleton. About 50% water by weight of the PMC (including encapsulated perfume and/ or malodor reducing composition) is assumed.
(6) Diethylene triamine pentaacetic acid
(7) 19% active aqueous solution of 1,2 Benzisothiazolin-3-one (BIT) in dipropylene glycol and water available from Dow Chemical under the trade name Koralone B-119
(8) Silicone antifoam agent available from Dow Corning ® under the trade name DC2310.
(9) Polyethylene imines available from BASF under the trade name Lupasol ®.
(10) Polydimethylsiloxane emulsion from Dow Corning ® under the trade name DC346.
(11) Non-ionic such as TWEEN 20 ™ or cationic surfactant as Berol 648 and Ethoquad ® C 25 from Akzo Nobel.
(12) Organosiloxane polymer condensate made by reacting hexamethylenediisocyanate (HDI), and a, w silicone diol and 1,3-propanediamine, N'-(3-(dimethylamino)propyl)-N,N-dimethyl- Jeffcat Z130) or N-(3-dimethylaminopropyl)-N, Ndiisopropanolamine (Jeffcat ZR50) commercially available from Wacker Silicones, Munich, Germany.
(13) Fineoxocol ® 180 from Nissan Chemical Co.
(14) Isofol ® 16 from Sasol.
**For example PGE

Examples 11.1 to 11.4 are made by combining the molten fabric softener active with the front-end stability agent to form a first mixture. This first mixture is combined with water and hydrochloric acid using a high shear mixing device to form a second mixture. The adjunct ingredients are combined with the second mixture using low shear mixing to form the fabric enhancing formula.

Examples 11.1 through 11.4 are used by dosing 10 to 60 g of the formula into the rinse liquor for example via dispensing into a clothes washing machine. Clothes are dried on a line or in an automated clothes dryer. The fabrics treated with these formulas have improved feel and scent and are effective at reducing malodor on the clothing.

Example 12.1-12.2 - Spray-Dried Laundry Detergent Powder composition and Process of Making it

An example of a Spray-Dried Laundry Detergent Powder composition prepared with malodor reduction composition, according to the compositions shown in Example 1.

| Compositions | Slurry 12.1 | Dried Powder 12.1 | Slurry 12.2 | Dried Powder 12.2 |
|---|---|---|---|---|
| | w/w% | w/w% | w/w% | w/w% |
| Linear alkyl benzene sulfonate | 10.6 | 15.8 | 21.3 | 35.7 |
| Acrylate/ maleate copolymer | 4.6 | 6.8 | 9.4 | 14.2 |

(continued)

| Compositions | Slurry 12.1 | Dried Powder 12.1 | Slurry 12.2 | Dried Powder 12.2 |
|---|---|---|---|---|
| | | | | |
| | w/w% | w/w% | w/w% | w/w% |
| Ethylenediame disuccinic acid and /or Hydroxyethane dimethylene phosphonic acid | 1.4 | 2.1 | 1.7 | 2.9 |
| Sodium carbonate | 19.4 | 26.5 | 18.8 | 29.9 |
| Sodium sulfate | 28.6 | 42.4 | --- | --- |
| Carboxy methyl cellulose polymer | --- | --- | 4.3 | 7.1 |
| Miscellaneous, such as magnesium sulfate, brightener and one or more stabilizers | 1.4 | 2.2 | 2.5 | 4.2 |
| Free (Neat) Perfume (spray-on) | --- | 1.7 | --- | 2.4 |
| Free (Neat) malodor reduction composition (spray-on) | --- | 0.5 | --- | 0.6 |
| Water | 34.0 | 2.0 | 42.0 | 3.0 |

Preparation of Spray-Dried Laundry Detergent Powders.

Aqueous slurry 12.1 having the composition as described above is prepared having a moisture content of 34.0%. Any ingredient added above in liquid form is heated to 70° C., such that the aqueous slurry is never at a temperature below 70° C. At the end of preparation, the aqueous slurry is heated to 80° C. and pumped under pressure ($5 \times 10^6$Nm$^{-2}$), into a counter current spray-drying tower with an air inlet temperature of from 290° C. The aqueous slurry is atomized and the atomized slurry is dried to produce a solid mixture, which is then cooled and sieved to remove oversize material (>1.8 mm) to form a spray-dried powder, which is free-flowing. Fine material (<0.15 mm) is elutriated with the exhaust the exhaust air in the spray-drying tower and collected in a post tower containment system. The spray-dried powder 12.1 has a moisture content of 2.0 wt %, a bulk density of 310 g/l and a particle size distribution such that greater than 90 wt % of the spray-dried powder has a particle size of from 150 to 710 micrometers. The composition of the spray-dried powder 12.1 is listed in the table above. Perfume and malodor reduction composition are sprayed onto the composition following the spray dry procedure.

Aqueous slurry 12.2 having the composition as described above is prepared having a moisture content of 42.0%. Any ingredient added above in liquid form is heated to 70° C., such that the aqueous slurry is never at a temperature below 70° C. At the end of preparation, the aqueous slurry is heated to 85° C. and pumped under pressure (from $6.5 \times 10^6$Nm$^{-2}$), into a counter current spray-drying tower with an air inlet temperature of from 275° C. The aqueous slurry is atomized and the atomized slurry is dried to produce a solid mixture, which is then cooled and sieved to remove oversize material (>1.8 mm) to form a spray-dried powder 12.2, which is free-flowing. Fine material (<0.15 mm) is elutriated with the exhaust the exhaust air in the spray-drying tower and collected in a post tower containment system. The spray-dried powder has a moisture content of 3.0 wt %, a bulk density of 250 g/l and a particle size distribution such that greater than 90 wt % of the spray-dried powder has a particle size of from 150 to 710 micrometers. The composition of the spray-dried powder is given in the table above. Perfume and malodor reduction composition are sprayed onto the composition after the spray dry process.

Example 13: Plastic film Example with Malodor Reducing Composition

A plastic film is treated with a Malodor Reduction Composition disclosed in the present specification. Said treatment is accomplished by spraying said Malodor Reduction Composition on the film. Said Malodor Reduction Composition is applied to at a level of 0.01% to 5% based on weight of the film. The Malodor Reduction Composition is found on the surface and may also be absorbed into the plastic film.

Example 14: Diaper example with Malodor reducing composition

A diaper article, is treated with a Malodor Reduction Composition disclosed in the present specification. The Malodor Reduction Composition is disposed on one or more areas such as:

- the garment-facing side;

- the body-facing side of the topsheet;

- the absorbent core;

- the body-facing side of the backsheet.

The Malodor Reduction Composition may also be disposed on the garment-facing side of the absorbent core and/or on other components such as the garment facing side of a nonwoven dusting layer or body-facing side of a secondary topsheet or acquisition layer. In some embodiments, a topsheet or an acquisition layer may comprise a tissue layer and/or a nonwoven layer, and the malodor reduction composition may be disposed on either the tissue layer or the nonwoven layer. As discussed below, the Malodor Reduction Composition may be in the absorbent article via incorporation into an adhesive. In some embodiments, the Malodor Reduction Composition may be incorporated into the absorbent article via a lotion that is applied to a substrate component of the article. In some embodiments, the malodor reduction composition may be incorporated into the absorbent article via an elastomeric film. In some embodiments, the malodor reduction composition may be in an article by having been incorporated into the making of an article component, including, but not limited to, a film, a nonwoven, AGM, elastics, and/or ink.

Example 16.1-16.5 Deodorant example with Malodor reducing composition.

An example of Deodorant compositions prepared with malodor reduction composition, according to the compositions shown in Example 1.

| Ingredient | 16.1 | 16.2 | 16.3 | 16.4 | 16.5 |
|---|---|---|---|---|---|
| Product Form | Solid Deodorant Control | Solid Deodorant | Solid Deodorant | Solid Deodorant | Aerosol Deodorant or Body Spray |
| dipropylene glycol | 48 | 48 | 20 | 30 | 20 |
| propylene glycol | 19.3 | 19.3 | 22 | - | - |
| tripopylene glycol | - | - | 25 | - | - |
| Glycerine | - | - | - | 10 | - |
| PEG-8 | - | - | - | 20 | - |
| Propylene Glycol 3 Myristyl Ether | 1.4 | 1.4 | - | - | - |
| ethanol | - | - | - | - | QS |
| Water | QS | QS | QS | QS | - |
| sodium stearate | 5.4 | 5.4 | 5.5 | 5.5 | - |
| tetra sodium EDTA | 0.5 | 0.5 | 0.05 | 0.05 | - |
| sodium hydroxide | - | - | 0.04 | 0.04 | - |
| triclosan | - | - | 0.3 | 0.3 | - |
| Neat Perfume | 2.8 | 2.8 | 2 | 1.5 | 1.5 |
| Malodor reducing composition | - | 0.7 | 1.0 | 0.5 | 0.35 |
| Blue 1 | 0.0009 | 0.0009 | - | - | - |
| Propellant (1,1 difluoroethane) | - | - | - | - | 40 |
| QS - Indicates that this material is used to bring the total to 100%. | | | | | |

Examples 17.1-17.3: Body wash with Malodor reducing composition

An example of Body Wash compositions prepared with malodor reduction composition, according to the compositions shown in Example 1.

|  | 17.1 Body Wash | 17.2 Body Wash | 17.3 Body Wash |
|---|---|---|---|
| Sodium Laureth-3 Sulfate (as 28% active) | 27.85% | 27.85% | 27.85% |
| Water | Q.S. | Q.S. | Q.S. |
| Sodium Lauryl Sulfate (as 29% active) | 10.34 | 10.34 | 10.34 |
| Cocamidopropyl Betaine B (30% active) | 4.01 | 4.01 | 4.01 |
| Citric Acid | 0.18 | 0.18 | 0.18 |
| Sodium Benzoate | 0.3 | 0.3 | 0.3 |
| Disodium EDTA | 0.12 | 0.12 | 0.12 |
| Methylchloroisothiazolinone/Methy lisothiazolinone | 0.04 | 0.04 | 0.04 |
| Sodium Chloride | 2.35 | 1.7 | 1.6 |
| Neat Perfume | 1.25 | 1 | 2 |
| Malodor reducing composition | 0.25 | 0.175 | 0.25 |
| QS - indicates that this material is used to bring the total to 100%. | | | |

Examples 18.1-18.6: Antiperspirant examples with Malodor reducing composition

An example of Antiperspirant compositions prepared with malodor reduction composition, according to the compositions shown in Example 1.

|  | 18.1 Invisible Solid | 18.2 Invisible Solid | 18.3 Invisible Solid | 18.4 Soft Solid | 18.5 Soft Solid | 18.6 Soft Solid |
|---|---|---|---|---|---|---|
| Aluminum Zirconium Trichlorohydrex Glycine Powder | 24 | 24 | 24 | 26.5 | 26.5 | 26.5 |
| Cyclopentasiloxane | Q.S | Q.S. | Q.S. | Q.S. | Q.S. | Q.S. |
| Dimethicone | - | - | - | 5 | 5 | 5 |
| CO-1897 Stearyl Alcohol NF | 14 | 14 | 14 | - | - | - |
| Hydrogenated Castor Oil MP80 Deodorized | 3.85 | 3.85 | 3.85 | - | - | - |
| Behenyl Alcohol | 0.2 | 0.2 | 0.2 | - | - | - |
| Tribehenin | - | - | - | 4.5 | 4.5 | 4.5 |
| C 18 - 36 acid triglyceride | - | - | - | 1.125 | 1.125 | 1.125 |
| C12-15 Alkyl Benzoate | 9.5 | 9.5 | 5 | - | - | - |
| PPG-14 Butyl Ether | 6.5 | 6.5 | - | 0.5 | 0.5 | 0.5 |
| Phenyl Trimethicone | 3 | - | 3 | - | - | - |
| White Petrolatum | 3 | - | - | 3 | 3 | 3 |
| Mineral Oil | 1.0 | 1.0 | 1.0 | - | - | - |
| Free (Neat) Perfume | 1.0 | 0.75 | 2.0 | 0.75 | 1.0 | 1.25 |
| Malodor reducing composition | 0.25 | - | 0.35 | 0.175 | 0.25 | 0.1 |

(continued)

|  | 18.1 Invisible Solid | 18.2Invisible Solid | 18.3Invisible Solid | 18.4Soft Solid | 18.5Soft Solid | 18.6Soft Solid |
|---|---|---|---|---|---|---|
| Beta-Cyclodextrin complexed with Malodor reducing composition | - | 3.0 | - | - | - | 3.0 |
| Talc Imperial 250 USP | 3.0 | 3.0 | 3.0 | - | - | - |
| Polyacrylate Microcapsule loaded with Malodor reducing composition | - | - | 1.9 | - | - | - |
| QS - Indicates that this material is used to bring the total to 100%. | | | | | | |

Example 19.1-19.4: Dish cleansing composition examples with Malodor reducing composition

An example of Dish cleaning compositions prepared with malodor reduction composition, according to the compositions shown in Example 1.

|  | 19.1 | 19.2 | 19.3 | 19.4 |
|---|---|---|---|---|
| Alkyl $C_{10-14}$ Ethoxy Sulphate (AE0.6S) | 26.9 | - | - | 25.7 |
| Alkyl $C_{10-14}$ Ethoxy Sulphate (AE2S) | - | 18.7 | 26.9 | - |
| Sodium alkyl benzene sulfonate | - | 8.0 | - | - |
| C12-14 dimethyl amine oxide | 6.1 | - | - | 4.1 |
| Cocamido propyl betaine | - | 4.5 | 6.8 | 3.2 |
| Branched Nonionic: 3-propyl heptanol EO8 | 1.0 | 0.8 | - | - |
| PEI600-EO10-PO7 block polymer | - | - | 0.8 | - |
| Perfume | 0.15-0.3 | 0.15-0.3 | 0.15-0.3 | 0.15-0.3 |
| Malodor Reducing Composition | 0.06-0.15 | 0.06-0.15 | 0.06-0.15 | 0.06-0.15 |
| Ethanol | 4.0 | 5.0 | 3.0 | 3.0 |
| Polypropylene glycol MW2000 | 1.1 | 0.8 | 1.1 | 1.1 |
| Sodium Chloride | 1.3 | 0.8 | 1.3 | 0.5 |
| Minors* and water | to balance up to 100% | | | |

Examples 20.1-20.4 - Heavy Duty Liquid Composition

A HDL-Heavy Duty Liquid composition is prepared with malodor reduction composition, according to the compositions shown in Example 1.

|  | Example 20.1 | Example 20.2 | Example 20.3 | Example 20.4 |
|---|---|---|---|---|
| Ingredient | WT% Active | WT% Active | WT% Active | WT% Active |
| $AE_{1.8}S$ | 16 | 16 | 12 | 8 |
| $C_{11.8}$ linear alkyl benzene sulfonic acid | 3 | 3 | 8 | -- |
| HSAS[1] | 14 | 14 | 0 | 22 |
| C24 alcohol, EO9 | 2 | 2 | 1 | 2 |
| Citric Acid | 1 | 1 | 2 | 2 |

(continued)

| Ingredient | Example 20.1 WT% Active | Example 20.2 WT% Active | Example 20.3 WT% Active | Example 20.4 WT% Active |
|---|---|---|---|---|
| Lactic Acid | --- | 6 | --- | --- |
| $C_{12}$-$C_{18}$ Fatty Acid | 2 | 1 | 1 | 3 |
| Protease (55.3 mg/g) | 2 | 2 | 2 | 2 |
| Amylase (25.4mg/g) | 0.7 | 0.7 | 0.7 | 0.7 |
| Borax | 3.6 | 3.6 | 3.6 | 3.6 |
| Calcium Formate | 0.2 | 0.2 | 0.2 | 0.2 |
| Polyethyleneimine 600, EO20 | 1.6 | 1.6 | --- | 1.6 |
| Polyethyleneimine 600, EO24, PO16 | 1.6 | --- | 2.0 | 1.6 |
| DTPA[2] | 0.3 | 0.3 | 0.3 | 0.3 |
| Tiron®[3] | 0.8 | 0.8 | 0.8 | 0.8 |
| Optical Brightener | 0.3 | 0.3 | 0.3 | 0.3 |
| NaOH | 4 | 9 | 4 | 9 |
| Na Cumene Sulfonate | 1 | 1 | 1 | 1 |
| Na Formate | 0.2 | 0.2 | 0.2 | 0.2 |
| Aesthetics Dye | 0.03 - 1 | 0.03 - 1 | 0.03 - 1 | 0.03 - 1 |
| Perfume | 0.5 - 3 | 0.5 - 3 | 0.5 - 3 | 0.5 - 3 |
| Malodor Reducing Composition | 0.1-1 | 0.1 - 1 | 0.1 - 1 | 0.1 - 1 |
| Water and Solvent | Balance | Balance | Balance | Balance |
| pH | 5 | 8 | 5 | 8 |

The resulting heavy duty liquid product when used to wash articles of clothing is effective at reducing malodor on the washed clothing.

Examples 21.1-21.3 - Heavy Duty Liquid Composition

Other examples of HDL-Heavy Duty Liquid composition prepared with malodor reduction composition, according to the compositions shown in Example 1.

| Ingredient | Example 21.1 WT% Active | Example 21.2 WT% Active | Example 21.3 WT% Active |
|---|---|---|---|
| $AE_{1.8}S$ | 14 | 8 | 11 |
| Amine Oxide | 2 | --- | --- |
| $C_{11.8}$ linear alkyl benzene sulfonic acid | 10% | --- | --- |
| HSAS[1] | --- | 20 | 15 |
| C24 alcohol, EO9 | 1 - 2 | 1 - 2 | 1 - 2 |
| Citric Acid | 0.5 - 3 | 0.5 - 3 | 0.5 - 3 |
| $C_{12}$-$C_{18}$ Fatty Acid | 1 - 2 | 1 - 2 | 1 - 2 |
| Protease (55.3 mg/g) | 0.013 - 0.8 | 0.013 - 0.8 | 0.013 - 0.8 |
| Amylase (25.4mg/g) | 0.02 - 0.7 | 0.02 - 0.7 | 0.02 - 0.7 |

(continued)

| Ingredient | Example 21.1 WT% Active | Example 21.2 WT% Active | Example 21.3 WT% Active |
|---|---|---|---|
| Borax | 2 - 8 | 2 - 8 | 2 - 8 |
| Calcium Formate | 0.2 | 0.2 | 0.2 |
| Carezyme 5.0L | 0.01 - 0.5 | --- | 0.01 - 0.5 |
| Polyethyleneimine 600, EO20 | 0.5 - 3 | 0.5 - 3 | 0.5 - 3 |
| Polyethyleneimine 600, EO24, PO16 | 0.5 - 3 | --- | 0.5 - 3 |
| DTPA2 | 0.1 - 1 | 0.1 - 1 | 0.1 - 1 |
| Tiron®[3] | 0.1 - 2 | 0.1 - 2 | 0.1 - 2 |
| Optical Brightener | 0.1 - 1 | 0.1 - 1 | 0.1 - 1 |
| MEA - Hydrogenated Castor Oil | | 0.5 - 3 | 0.5 - 3 |
| NaOH | 0.1 - 1 | 0.1 - 1 | 0.1 - 1 |
| Na Cumene Sulfonate | 1.1 | --- | --- |
| Na Formate | 0.2 | 0.2 | 0.2 |
| Dye | 0.1 | 0.1 | 0.1 |
| Malodor Reducing Composition | 0.15 - 1.0 | --- | 0.15 - 1.0 |
| Encapsulated Malodor Reducing[4] | --- | 0.3 - 3 | --- |
| Perfume | 0.5-2 | 0.5 - 2 | --- |
| Encapsulated Perfume[4] | --- | --- | 1.5 - 3 |
| Water and Solvent | Balance | Balance | Balance |

1. HSAS is secondary alkyl sulfate, acid form
2. DTPA is diethylene triamine pentaacetic acid (DTPA)
3. Tiron is 4,5-Dihydroxy-1,3-benzenedisulfonic acid disodium salt monohydrate
4. Encapsulated perfume and encapsulated malodor reducing composition is a slurry. One non-limiting example of an encapsulate is a friable microcapsule with a urea-formaldehyde shell from Appvion-Encapsys. About 50% water by weight of the PMC (including encapsulated perfume) is assumed.

The resulting heavy duty liquid product when used to wash articles of clothing is effective at reducing malodor on the washed clothing.

Examples 22.1-22.3 - Fabric Enhancer Solid Particle or Bead Composition

An example of a Fabric Enhancer bead composition prepared with malodor reduction composition, according to the compositions shown in Example 1.

| Ingredients | Example 22.1 Active Wt% | Example 22.2 Active Wt% | Example 22.3 Active Wt% |
|---|---|---|---|
| PEG 8000 | 80 - 90 | 80-90 | 80 - 90 |
| Free (Neat) Perfume | 3.5 - 6 | 4 - 6 | 3 - 5 |
| Malodor reducing composition | 0.5 - 2 | 0.5 - 2 | 0.5 - 2 |
| Encapsulated Perfume[1] | 0 - 2 | 0 - 2 | 2 - 3 |
| Encapsulated Malodor Reducing Composition[1] | --- | 0.5 - 2 | --- |
| Dipropylene Glycol | 0-6 | 0-5 | 0-4.5 |

(continued)

|  | Example 22.1 | Example 22.2 | Example 22.3 |
|---|---|---|---|
| **Ingredients** | **Active Wt%** | **Active Wt%** | **Active Wt%** |
| Aesthetics Dye | 0.015 | 0.015 | 0.015 |

1. Encapsulated perfume and encapsulated malodor reducing composition is a slurry. One non-limiting example of an encapsulate is a friable microcapsule with a urea-formaldehyde shell from Appvion-Encapsys. About 50% water by weight of the PMC (including encapsulated perfume) is assumed.

The resulting Fabric enhancing bead product when added to an automatic washing machine is effective at reducing malodor on the clothing.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A composition comprising a malodor reduction material is selected from the group consisting of 3,5-dimethyladamantane-1-carboxylic acid; (1R, 6S)-6-(methoxycarbonyl)cyclohex-3-ene-1-carboxylic acid; (E)-1,1,4,4-tetramethoxybut-2-ene;
dimethyl (4R,5R)-2,2-dimethyl-1,3-dioxolane-4,5-dicarboxylate; 1,2,3,4,5,6-hexamethylbenzene; dimethyl (1R,2S)-cyclohex-4-ene-1,2-dicarboxylate and mixtures thereof.

2. A composition comprising, based on total composition weight, from 0.0001% to 100% of a malodor reduction composition according to Claim 2 and mixtures thereof, and an adjunct material, said composition being a consumer product.

3. A plastic film said plastic film comprising LLDPE, LDPE, HDPE, and/or compostable film and a malodor reduction composition according to Claim 1 and mixtures thereof.

4. A method of controlling malodors comprising contacting a material comprising a malodor with a composition according to Claim 1 and mixtures thereof.

Europäisches
Patentamt

European
Patent Office

Office européen
des brevets

# EUROPEAN SEARCH REPORT

Application Number

EP 17 18 0038

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 110 852 A (GOGOLEWSKI SYLWESTER [CH] ET AL) 5 May 1992 (1992-05-05) * claim 1 * | 1,2,4 | INV. A61L9/01 C08L23/06 |
| X | US 2016/158377 A1 (ACKLER SCOTT L [US] ET AL) 9 June 2016 (2016-06-09) * paragraph [0573] * | 1,2,4 | |
| X | US 2008/228009 A1 (RICHTER INGO [DE] ET AL) 18 September 2008 (2008-09-18) * example 1 * | 1,2,4 | |
| X | Anonymous: "(1S,2R)-1-Methyl cis-1,2,3,6-tetrahydrophthalate >=97.0% (sum of enantiomers, HPLC) ¦ Sigma-Aldrich", , 31 December 2016 (2016-12-31), XP055400174, Retrieved from the Internet: URL:http://www.sigmaaldrich.com/catalog/product/aldrich/87462?lang=en ion=NL [retrieved on 2017-08-22] * the whole document * | 1,2,4 | TECHNICAL FIELDS SEARCHED (IPC)<br>A61L C08L |
| X | Anonymous: "Dimethyl (1R,2S)-4-cyclohexene-1,2-dicarboxylate ¦ C10H1404 ¦ ChemSpider", , 31 December 2016 (2016-12-31), XP055400182, Retrieved from the Internet: URL:http://www.chemspider.com/Chemical-Structure.9042564.html [retrieved on 2017-08-22] * the whole document * | 1,2,4 | |

-/--

The present search report has been drawn up for all claims

| Place of search | Date of completion of the search | Examiner |
|---|---|---|
| Munich | 23 August 2017 | Bertrand, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 17 18 0038

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/266642 A1 (HOLLINGSHEAD JUDITH ANN [US] ET AL) 10 October 2013 (2013-10-10) * claims 17-21 * ----- | 3 | |
| X | Anonymous: "DIETHYL (4R,5R)-2,2-DIMETHYL-1,3-DIOXOLANE-4,5-DICARBOXYLATE AldrichCPR ¦ Sigma-Aldrich", , 31 December 2016 (2016-12-31), XP055400162, Retrieved from the Internet: URL:http://www.sigmaaldrich.com/catalog/product/aldrich/t282278?lang=en ion=NL [retrieved on 2017-08-22] * the whole document * ----- | 1,2,4 | |

TECHNICAL FIELDS
SEARCHED      (IPC)

The present search report has been drawn up for all claims

| Place of search | Date of completion of the search | Examiner |
|---|---|---|
| Munich | 23 August 2017 | Bertrand, Samuel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

EP 3 424 539 A1

## ANNEX TO THE EUROPEAN SEARCH REPORT
## ON EUROPEAN PATENT APPLICATION NO.

EP 17 18 0038

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2017

| Patent document cited in search report | | Publication date | Patent family member(s) | | Publication date |
|---|---|---|---|---|---|
| US 5110852 | A | 05-05-1992 | NONE | | |
| US 2016158377 | A1 | 09-06-2016 | AU 2015360621 A1 | | 29-06-2017 |
| | | | US 2016158377 A1 | | 09-06-2016 |
| | | | WO 2016094517 A1 | | 16-06-2016 |
| US 2008228009 | A1 | 18-09-2008 | CA 2617556 A1 | | 08-02-2007 |
| | | | CN 101233263 A | | 30-07-2008 |
| | | | DE 102005036687 A1 | | 08-02-2007 |
| | | | EP 1913178 A1 | | 23-04-2008 |
| | | | JP 2009503266 A | | 29-01-2009 |
| | | | KR 20080044257 A | | 20-05-2008 |
| | | | US 2008228009 A1 | | 18-09-2008 |
| | | | WO 2007014932 A1 | | 08-02-2007 |
| US 2013266642 | A1 | 10-10-2013 | AU 2013246305 A1 | | 30-10-2014 |
| | | | AU 2016204131 A1 | | 14-07-2016 |
| | | | CA 2869877 A1 | | 17-10-2013 |
| | | | CN 104379714 A | | 25-02-2015 |
| | | | EP 2836579 A2 | | 18-02-2015 |
| | | | JP 6139660 B2 | | 31-05-2017 |
| | | | JP 2015520619 A | | 23-07-2015 |
| | | | KR 20140142282 A | | 11-12-2014 |
| | | | US 2013266642 A1 | | 10-10-2013 |
| | | | WO 2013154899 A2 | | 17-10-2013 |

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070275866 A1
- US PA20050003980 A1
- US 6413920 B1
- US PN4137180 A